# EUROPEAN PATENT APPLICATION

(11) **EP 4 671 727 A1**
(43) Date of publication of application: **31.12.2025**
(21) Application number: 24184219.4
(22) Date of filing: 25.06.2024
(51) Int. Cl.: G01M 17/02, G01N 33/44, G01N 33/36, B60C 99/00, G01N 3/30

(54) **METHOD OF CORRELATING CARCASS FABRIC PROPERTIES WITH TIRE PROPERTIES**

(71) Applicant: The Goodyear Tire & Rubber Company, Akron, OH 44316 (US)
(72) Inventor: BUCHOLD, Philipp Constantin, L-7519 Mersch (LU); VANDERHAEGHEN, Julie, B-6600 Bastogne (BE); LIONETTI, Robert Edward, L-7227 Bereldange (LU); BONNET, Gilles, L-9176 Niederfeulen (LU); GILLICK, James Gregory, Akron, 44313 (US); FEHL, Helmut Wolfgang, D-36381 Schluchtern (DE)
(74) Representative: Kutsch, Bernd

(57) **Abstract**

A method of correlating fabric properties with tire properties is disclosed. The method comprises the steps: providing a set of different types of fabrics; determining a magnitude of a measure of a strength of each type of fabric of the set of different types of fabrics; providing a plurality of tires (1), wherein each tire of the plurality of tires comprises one type of fabric of the set of different types of fabrics as carcass fabric (3) of the tire (1); carrying out mechanical impacts of different magnitude on the surface of a shoulder region (7) of each tire of the plurality of tires; checking each tire of the plurality of tires for a presence of a visual damage indicator at an outer surface of one or more of a sidewall region (6) and the shoulder region (7) of the tire upon carrying out one of the mechanical impacts; determining for each tire of the plurality of tires a magnitude of a measure of a mechanical impact causing the presence of the visual damage indicator; and assigning the magnitude of the measure of the mechanical impact causing the presence of the visual damage indicator of each tire of the plurality of tires to the magnitude of the measure of the strength of the type of fabric comprised in the respective tire, so as to obtain for the set of different types of fabrics value pairs comprising the magnitude of the measure of the strength of one type of fabric and the magnitude of the measure of the mechanical impact causing the presence of the visual damage indicator for the tire comprising said one type of fabric.

## Description

### Field of the Invention

The present invention is directed to a method of correlating fabric properties, such as carcass fabric properties, to tire properties. Moreover, the present invention is directed to a method of developing a tire, particularly comprising the method of correlating fabric properties with tire properties.

### Background of the Invention

Designing or developing new tires, such as pneumatic radial passenger car tires, typically involves building and physically testing a significant number of test tires which requires time and resources. Thus, it would be desirable to provide methods which allow for less test tire builds and/or less physical tire tests, resulting in faster development processes and requiring less resources, such as raw materials and energy. While improvements have been made in this field over the past years, significant room for improvement remains.

### Summary of the Invention

The invention relates to a method in accordance with claim 1.

Dependent claims refer to preferred embodiments of the invention.

A first preferred aspect of the present invention is directed to a method of correlating fabric properties with tire properties comprising the steps of providing a set of different types of fabrics, determining a magnitude of a measure of a strength of each type of fabric of the set of different types of fabrics, and providing a plurality of tires, wherein each tire of the plurality of tires comprises one type of fabric of the set of different types of fabrics as carcass fabric of the tire. Moreover, the method comprises carrying out mechanical impacts of different magnitude on the surface of a shoulder region of each tire of the plurality of tires, and checking each tire of the plurality of tires for a presence of a visual damage indicator at an outer surface of one or more of a sidewall region and the shoulder region of the tire upon carrying out one of the mechanical impacts. Furthermore, the method comprises the steps of determining for each tire of the plurality of tires a magnitude of a measure of a mechanical impact causing the presence of the visual damage indicator, and assigning the magnitude of the measure of the mechanical impact causing the presence of the visual damage indicator of each tire of the plurality of the tires to the magnitude of the measure of the strength of the type of fabric comprised in the respective tire, so as to obtain for the set of different types of fabrics value pairs comprising i) the magnitude of the measure of the strength of one type of fabric and ii) the magnitude of the measure of the impact causing the presence of the visual damage indicator for the tire comprising said one type of fabric. Still in accordance with the first aspect, the method comprises the step of determining a correlation based on the obtained value pairs.

A second preferred aspect of the present invention is directed to a method of developing a tire. This method comprises at least the method steps according to the first aspect, and the further steps of providing a further type of fabric, which is different from the types of fabrics of said set of different types of fabrics; and determining a magnitude of the measure of the strength of the further type of fabric. Furthermore, the method of developing a tire comprises the steps of providing an estimated magnitude of the measure of a mechanical impact causing the presence of the visual damage indicator based on the determined magnitude of the measure of the strength of the further type of fabric and said determined correlation.

Each tire of the plurality of tires is preferably one or more of a pneumatic tire, a passenger car tire, and a radial tire.

Each tire of the plurality of tires preferably has a tread portion and a pair of bead portions, wherein the carcass fabric extends from one bead portion of the pair of bead portions to another bead portion of the pair of bead portions, and at least one belt arranged radially between the carcass fabric and the tread portion in a crown region of the tire.

The mechanical impacts are preferably carried out in accordance with standard GB/T 38528-2020. The threshold value is preferably the minimum breaking energy as defined in the standard.

### Brief Description of the Drawings

The invention will be described by way of example and with reference to the accompanying drawings in which:
FIG. 1 is a schematic cross section of a pneumatic tire comprising a carcass fabric;
FIG. 2 is a schematic perspective view of a portion of a fabric;
FIG. 3 is a graphic representation of value pairs and interpolated graphs based on fabric breaking strength per 2.54 cm against average kinetic energy causing the presence of a visual tire damage indicator;
FIG. 4a is a schematic sideview of a striker pendulum test arrangement for striking a shoulder region of a tire; and
FIG. 4b is a schematic plan view of the striker pendulum test arrangement of FIG. 4a, from a different perspective.

### Detailed Description of Preferred Embodiments the Invention

A first preferred embodiment is directed to the method of correlating fabric properties with tire properties comprising the steps of providing a set of different types of fabrics; determining a magnitude of a measure of a strength of each type of fabric of the set of different types of fabrics; and providing a plurality of tires, wherein each tire of the plurality of tires comprises one type of fabric of the set of different types of fabrics as carcass fabric of the tire. Moreover, the method comprises carrying out mechanical impacts of different magnitude on the surface of a shoulder region of each tire of the plurality of tires, and checking each tire of the plurality of tires for a presence of a visual damage indicator (or tire damage indicator) at an outer surface (preferably, of one or more of a sidewall region and the shoulder region of the tire) upon, or after, carrying out one of the mechanical impacts (in other words, after each mechanical impact of said mechanical impacts). Furthermore, the method comprises the steps of determining for each tire a magnitude of a measure of a mechanical impact causing the presence of the visual damage indicator, and assigning the magnitude of the measure of the mechanical impact causing the presence of the visual damage indicator of each tire to the magnitude of the measure of the strength of the type of fabric comprised in the respective (mechanically impacted) tire, so as to obtain for the set of different types of fabrics value pairs comprising i) the magnitude of the measure of the strength of one type of fabric and ii) the magnitude of the measure of the impact causing the presence of the visual damage indicator for the (corresponding) tire comprising said (respective) one type of fabric. Still in accordance with the first aspect, the method comprises the step of determining a, preferably linear, correlation based on the obtained value pairs; or, in other words, between i) magnitudes of the measure of the strength of the types of fabrics and ii) magnitudes of the measure of the mechanical impacts causing the presence of the visual damage indicators, based on the obtained value pairs.

Such a determined correlation (such as a linear correlation or correlation function) has been found to be very helpful for the purpose of tire development. For instance, it has been found that such a correlation can be used to predict magnitudes of measures of mechanical impacts causing a visual damage indicator of a (virtual) tire comprising a further type of fabric of known (or estimated) fabric strength, or, in other words, of a known or estimated magnitude of the measure of the fabric strength. Thus, the determination of the correlation, or correlation function, allows to reduce the number of actual tire builds and/or to make predictions on the durability and/or robustness of a tire, particularly of its shoulder and/or sidewall region in view of mechanical impacts in the shoulder region of such a tire.

A preferred embodiment comprises carrying out the mechanical impacts includes mechanically impacting the tire in a first circumferential sector of its shoulder region with a first magnitude of mechanical impact, and optionally mechanically impacting the tire in a second circumferential sector of its shoulder region with a second magnitude of mechanical impact wherein the second magnitude is higher than the first magnitude; and/or mechanically impacting the tire in one or more further circumferential sectors of its shoulder region with one or more higher magnitudes of mechanical impact, preferably gradually increasing from circumferential sector to circumferential sector (e.g., a circumferentially adjacent and/or next sector) until the presence of the visual damage indicator is observed, optionally in one or more of the sidewall region and the shoulder region of the tire.

In a preferred embodiment, the visual damage indicator is one or more of a bulge, a crack, an exposed cord, an exposed portion of the (carcass) fabric, and an open splice. In a preferred embodiment, the visual damage indicator includes a bulge having a height of more than 1 mm. As mentioned herein, each tire is checked for the presence of such an indicator, preferably in the sidewall region and the shoulder region, and preferably at the same lateral side of the tire which is mechanically impacted. In particular, sidewall regions and/or shoulder regions are considered herein to extend in a circumferential direction of the tire. Optionally, in case the tire loses air, e.g., in view of a hole in the tire, no further impacts are carried out on the same tire. For instance, another tire comprising the same type of fabric can be provided and carrying out mechanical impacts may be resumed, such as starting with a lower magnitude of mechanical impact than the magnitude at which an air loss occurred.

In a preferred embodiment, the measure of the strength may comprise one or more of a breaking strength, work to break, elongation at break, such as determined according to ASTM D885, or equivalent.

In a preferred embodiment, the measure of the strength (of a fabric) is a breaking strength, as determined in accordance with ASTM D885, or equivalent. The breaking strength has been found to be the most desirable measure of fabric strength in view of very good coefficients of determination observed by the inventors.

In a preferred embodiment, the measure of a mechanical impact comprises one of a kinetic energy value of a mechanical impact means, and a parameter representative of a kinetic energy value of a mechanical impact means, particularly mechanically impacting the tire. It is emphasized that the present invention is not limited to a certain type of mechanical impact means. For instance, impact means optionally comprise one or more of a plunger, a piston, a striker, a striker pendulum. A magnitude of the measure of the mechanical impact causing the presence of the visual damage indicator can optionally also be mentioned herein as magnitude of the visual damage impact. A varying mass of said mechanical impact means may be a parameter representative of the kinetic energy (value or magnitude). In another example, an impact means may impact the tire with (e.g., gradually) increasing speed but same mass such that the varying speed may be such a parameter. In yet another example, the impact means may drop from a varying (e.g., gradually increasing) height so that the drop height may be a representative parameter. In case of a pendulum striker as impact means, a deflection angle (or, in other words, a striker angle, Sa, or drop angle) may be the parameter, or the height of deflection, or a potential energy of the pendulum striker. The terms striker pendulum and pendulum striker are used interchangeably herein. In addition, or alternatively, the magnitude and/or measure of the mechanical impact, particularly which is causing the presence of the visual damage indicator, may comprise an average value of multiple measurements. The term magnitude (or, alternatively, value) is used herein to quantify, for example, one or more of measures of strengths of fabrics, measures of mechanical impacts, and/or mechanical impacts.

In a preferred embodiment, the magnitude of a measure of a mechanical impact causing the presence of the visual damage indicator comprises one of a kinetic energy value of a mechanical impact means, and a parameter representative of a kinetic energy value of a mechanical impact means, when impacting the tire. Again, it is possible that such a value is an average value of multiple measurements, e.g., involving one or more of repeated mechanical impacts, refined increases of magnitudes of mechanical impacts, and gradually increasing magnitudes of the mechanical impact. Typically, mechanical impacts are carried out in circumferentially different, or in other words, spaced sectors of the tire. In other words, the mechanical impacts are not carried out multiple times at the same place or in the same circumferential sector. Optionally, each mechanical impact is carried out with a distance of at least 5 cm, preferably at least 10 cm, measured in the circumferential direction of the tire.

In a preferred embodiment, the correlation is a linear correlation, wherein the linear correlation optionally comprises one or more of a linear fit, a linear regression, a linear interpolation, and a linear extrapolation.

In a preferred embodiment, the mechanical impacts are carried out by a mechanical impact means, such as one of those mentioned herein above. In a preferred embodiment, the mechanical impacts are carried out by a striker pendulum, optionally in accordance with standard GB/T 38528-2020, or equivalent. As mentioned above, optionally, the measure of the magnitude of mechanical impact may comprise one or more of a deflection angle (or, in other words, drop angle) of the striker pendulum, a deflection height (or, in other words drop height) of the striker pendulum, and a kinetic energy of the striker pendulum, such as at the impact onto the shoulder region of the tire. As known to the person skilled in the art, it is possible to express and/or quantify the magnitude of mechanical impact with different variables, or measures.

In a preferred embodiment, each tire of the plurality of tires is one or more of a pneumatic tire, a passenger car tire, and a radial tire. Preferably, the tire is a cured tire, e.g., a sulfur cured tire.

In a preferred embodiment, each tire of the plurality of tires has a tread portion, a pair of bead portions, wherein the carcass fabric extends from one bead portion to another bead portion of the pair of bead portions, and at least one belt arranged radially between said carcass fabric and the tread portion in a crown region of the tire. Optionally, the tire comprises a carcass ply, extending from said one bead portion to said another bead portion, wherein the carcass ply comprises a rubber composition reinforced by the carcass fabric. In other words, the carcass fabric can be considered as a fabric (preferably, a textile fabric) reinforcing the carcass ply. Carcass plies reinforced by a carcass fabric are known in the art as such. The same applies to suitable rubber compositions, preferably sulfur-cured rubber compositions.

In a preferred embodiment, each tire of the plurality of tires is one or more of: a mono carcass ply tire; a tire comprising a carcass ply which comprises a rubber composition reinforced by (only) one type of the fabrics; and a tire having the same construction as the other tires of the plurality of tires, apart from the different type of fabric as carcass fabric. For instance, same construction includes herein having the same tire rim size, same tire width, same tire load index, and same tire aspect ratio.

In a preferred embodiment, the fabrics comprise one or more of a plurality of parallel textile cords; and one or more of polyester cords, polyamide cords, glass-fiber cords, carbon-fiber cords, and cords comprising plant-based material. In addition, or alternatively, the fabric or the fabrics are textile fabrics. Optionally, the cords and/or fabric(s) are dipped or coated, such as with adhesive dips or coatings, particularly for the adhesion to rubber composition material. For instance, resorcinol formaldehyde latex (RFL) may be used. While one or more of the above-mentioned cords are preferred, the fabrics optionally comprise steel cords.

In a preferred embodiment, the method comprises the steps of providing at least three different types of fabrics (preferably textile fabrics); determining, as the magnitude of the measure of the strength, a breaking strength of each fabric of the at least three different types of fabrics; providing at least three tires, wherein each tire of the at least three tires comprises a carcass ply comprising one type of fabric of the set of at least three different types of fabrics; providing for each tire, as the magnitude of the measure of a mechanical impact causing the presence of the visual damage indicator, one of a) a kinetic energy value, which results in an observation of the visual damage indicator, and b) a parameter representative of a kinetic energy value which results in an observation of the visual damage indicator, and wherein the visual damage indicator is preferably a bulge of more than 1 mm; and determining the, preferably linear, correlation, or correlation function, between i) breaking strengths of the at least three types of fabrics and ii) magnitudes of the measure of the mechanical impacts causing the presence of the visual damage indicators for the at least three tires, based on three obtained value pairs.

In a preferred embodiment, at least two of the breaking strengths differ by at least 1000 N/2.54 cm. In other words, the fabrics are chosen or provided in a manner that at least two of them have such a minimum difference in their breaking strengths. Such a choice helps to improve the accuracy/reliability of the correlation. The choice of at least two suitable fabrics can be carried out by a person skilled in the art based on one or more of common technical knowledge, cord or fabric supplier data, estimation, calculation, or testing.

In a preferred embodiment, the visual damage indicator is observed by one or more of: by a laser scanning device, by a camera, by a dial gauge, and by eye, wherein a dial gauge may also be a digital (dial) gauge. In one example, observation may be carried out as described in standard GB/T 38528-2020, or equivalent.

In a preferred embodiment, the method further comprises the steps of: providing a further type of fabric, which is different from the types of fabrics of the set of different types of fabrics; determining a magnitude of the measure of the strength of the further type of fabric; and, optionally, estimating a magnitude of a mechanical impact causing the presence of a visual damage indicator (e.g., of a tire, or virtual tire, comprising the further type of fabric as carcass fabric which has essentially the same, or the same, construction as other tires of the plurality of tires, apart from the different, further type of fabric as the carcass fabric) based on the determined magnitude of the measure of the strength of the further type of fabric and the correlation.

A second preferred embodiment of the invention is directed to a method of developing a tire (or simulating/predicting robustness of a tire, particularly in view of impacts to a shoulder of the tire). The method comprises the method steps according to the first aspect (and optionally one or more of its embodiments), and also comprises the further steps of: providing a further type of fabric, which is different from the types of fabrics of the set of different types of fabrics; determining a magnitude of the measure of the strength of the further type of fabric; and providing (or estimating) an estimated magnitude of a measure of a mechanical impact causing the presence of a visual damage indicator based on the determined magnitude of the measure of the strength of the further type of fabric and the, preferably linear, correlation. In other words, the provision of the estimated magnitude can be carried out without providing and/or building a physical tire. The estimation can also be considered as a prediction based on the correlation and the determined magnitude of a measure of a strength of the further type of fabric. The determination in relation to the strength of the further type of fabric does not need to involve an actual measurement but may also rely on known data such as of a fabric supplier or earlier fabric testing. The estimated magnitude may also be described as applying to a virtual tire comprising the further type of fabric as carcass fabric.

In a preferred embodiment, the method further comprises the steps of providing a threshold, or threshold value, for the magnitude of the measure of the mechanical impact; and checking whether the estimated magnitude is higher than the threshold. The threshold value can be chosen depending on the desired tire application. Typically, a higher threshold value means a higher robustness of the tire with respect to said mechanical impacts in the shoulder region of the tire. If a determined magnitude is higher than the threshold value, it is likely that an actually built tire would also pass the threshold in a respective test.

In a preferred embodiment, said mechanical impacts are carried out with a pendulum striker, preferably in accordance with standard GB/T 38528-2020, or equivalent.

In a preferred embodiment, said mechanical impacts are carried out in accordance with standard GB/T 38528-2020, and optionally said threshold value is the minimum breaking energy as defined in this standard.

While reference is made in some embodiments to standard GB/T 38528-2020, it is emphasized that other embodiments of the invention are not limited to the application of this standard and may be carried out under different conditions than mentioned therein, which is also within the scope of the present invention.

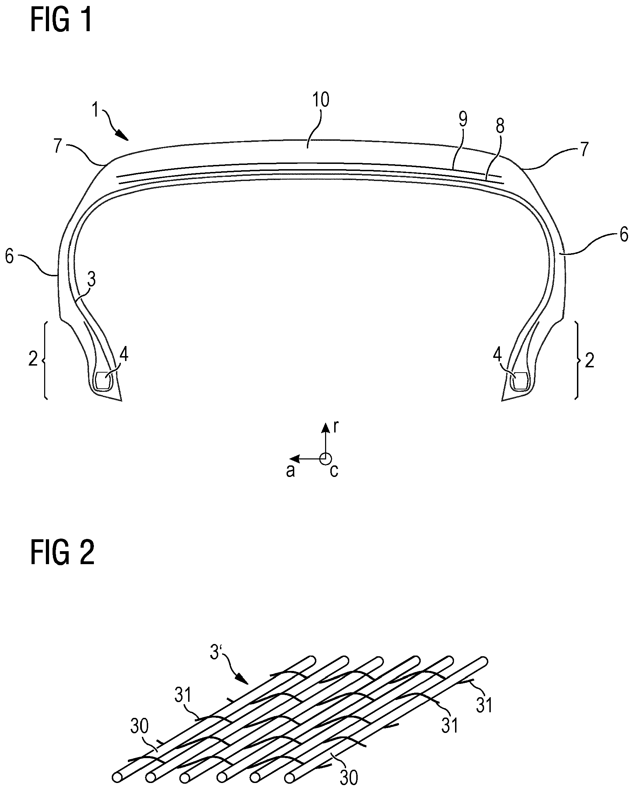

Figure 1 is a schematic cross section of a pneumatic tire 1 comprising a tread portion 10, a pair of bead portions 2 with respective beads 4, a pair of sidewalls 6, a carcass fabric 3 extending from bead portion 2 to bead portion 2 (and folded around each respective bead 4). Furthermore, the tire 1 comprises two belts 8, 9 which are arranged radially between the tread portion 10 and the carcass fabric 3 in a crown region of the tire 1. Moreover, the tire 1 has two shoulder regions 7 extending from the tread portion 10 of the tire to each respective sidewall 6.

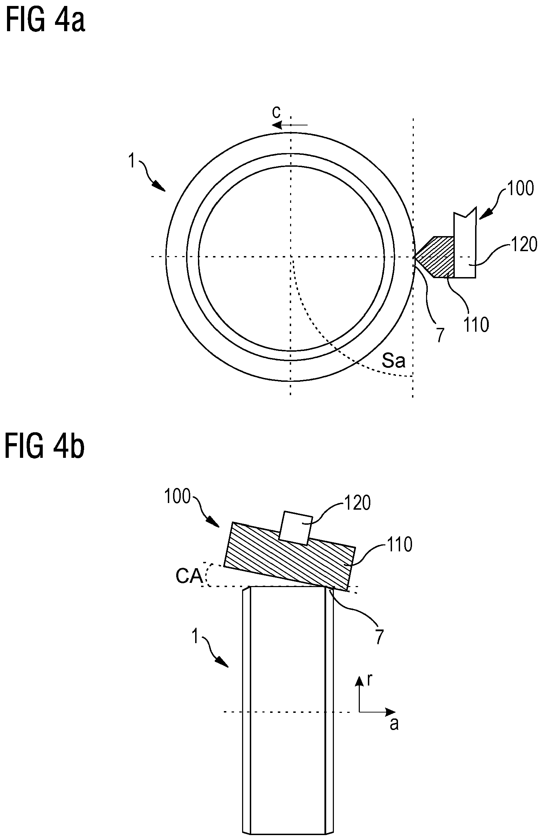

For the sake of better understanding, the radial direction r, the axial direction a, and the circumferential direction c of the tire 1 are indicated in Figure 1 and partially in Figures 4a and 4b. The axial direction a is parallel to the axis of rotation of the tire 1. The radial direction r is perpendicular to the axial direction a. The circumferential direction c is perpendicular to the axial direction and the radial direction, and, e.g., parallel to a circumferential centerline of the tire 1 or its equatorial plane. References to one of these directions are not necessarily limited to a specific orientation, unless indicated otherwise herein.

Figure 2 shows a portion of a fabric 3', similar to the carcass fabric 3 of the tire 1 indicated in Figure 1. In the depicted example, fabric 3' comprises a plurality of parallel textile cords 30, which may also be described as warp cords, and a plurality of cords 31 connecting the cords 30 transversely to their elongated extension. The cords 31 may also be described as weft cords. Optionally, fabric 3' can be described as a woven textile fabric 3'.

In accordance with preferred embodiments of the invention, different types of such fabrics are provided. In particular, different types of fabrics may comprise one or more of different cord materials (particularly different warp cord materials), different linear densities of the cords, different numbers of cords per 2.54 cm (in other words, ends per 2.54 cm, measured perpendicular to the elongated extension of the warp cords 30), and different cord constructions (e.g., including one or more of different twist factors, different lay lengths, different numbers of filaments and/or yarns forming the cords). In this context, it is emphasized that exact types of fabrics or its cords, or EPI ranges, are not within the main focus of the present invention.

Different cord materials may, for example, include polyester (e.g., PET), polyamide (e.g., Nylon^{™}), aromatic polyamide (aramid), glass-fiber cords, carbon-fiber cords, carbon-reinforced polyamide cords, carbon-reinforced polyester cords, and cords comprising plant-based material, such as including cotton, hemp, flax, sisal, and bast. Linear densities of cords could for instance range from 500 dtex to 10000 dtex. Cords may, for example, be monofilament cords or multifilament cords. Multifilament cords may, e.g., comprise multiple yarns or monofilaments. For instance, such yarns or monofilaments may be twisted with one another to form a cord. Optionally, some of those may be twisted as such before twisting them together to form the cord. In other examples, one or more filaments or yarns are centrally arranged within a cord and helically wrapped by one or more filaments or yarns. In many cases a multifilament cord comprises from 2 to 10 yarns or filaments. Typically, 5 to 40 cords or ends per 2.54 cm (EPI) are arranged in parallel in the fabric, such as within a range of 10 EPI to 40 EPI, preferably 10 EPI to 35 EPI.

In preferred embodiments of the methods of the present invention, a set of at least three different types of fabrics is provided, such as a set of four different types of textile fabrics.

In a further step, for each one of the three different types of textile fabrics, a breaking strength per 2.54 cm (BS/2.54 cm) is determined (preferably, in kN per 2.54 cm). There are various ways to determine such a breaking strength. In one preferred option, one or more warp cords are tested for their breaking strength in accordance with ASTM D885 (multiple tests including determination of averages is of course also possible) and then the value or average value obtained for one cord is multiplied by the number of cords per 2.54 cm of the fabric (i.e., the EPI value of the fabric), resulting in said breaking strength per 2.54 cm. In other words, a breaking strength per 2.54 cm value of a fabric can be considered as a breaking strength for (or per) the ends per 2.54 cm of a fabric.

In another step, at least three pneumatic radial tires are built, wherein each one of the tires comprises one of the three different types of fabric as carcass fabric, particularly in the tire's carcass ply, which is preferably a mono carcass ply tire, i.e., having only one carcass ply in a thickness of the tire's sidewall. Apart from the different carcass fabrics (or fabric types), the tires are essentially the same or equal, or, in other words, equally constructed. For instance, that means that they have the same tire components, diameter, width, load index, and section height. In other words, the tires essentially differ only in the different type of carcass ply.

Then each of the three tires is tested by applying mechanical impacts in its shoulder region. There are various potential mechanical impact procedures or means which are suitable for embodiments of the present invention.

According to the example of Figure 4a, the tire 1 is tested with a pendulum striker 100 having a striker 110 and striker arm 120. A tip of the striker 110 is arranged and adapted to mechanically impact the surface of the tire 1 in a shoulder region 7 of the tire 1. In particular, the pendulum striker 100, being rotatably mounted at an upper end of the striker arm 120, can be deflected by an angle, or striker angle (Sa) and released (or dropped) to mechanically impact the tire 1. The striker angle Sa may also be described as a drop angle or deflection angle. After a mechanical impact on the tire 1, the striker pendulum 100 is preferably stopped such as to avoid another hit at the same place or circumferential sector of the shoulder region of the tire 1. For a further mechanical impact, the tire 1 can be rotated about its axis of rotation, particularly, such that a further mechanical impact is carried out in a different circumferential sector of the tire 1. Preferably, the tire 1 is mechanically impacted from circumferential sector to circumferential sector with an increased magnitude of the impact, such as by increasing the striker angle Sa before a further impact. Thus, the striker 110 has a higher potential energy when being released which is converted into kinetic energy upon release / drop of the striker 110. In an embodiment, the striker angle Sa may be gradually increased by a value of 3° to 10°, such as by 5°, from impact to impact (and/or circumferential sector to circumferential sector), particularly, until the presence of a visual damage indicator is observed. In principle, the corresponding striker angle Sa can already be used for a correlation. However, preferably, in a further optional step, the striker angle Sa is reduced again to a value of at most the angle before the preceding gradual increase, and can be increased again, in further impacts from circumferential sector to circumferential sector, with smaller angular increases, e.g., within a range of 1° to 5°, such as with 2°. In particular, these smaller angular increases are smaller than the increment of the original angular increase, resulting in the first (or original) observation of the presence of the visual damage indicator. Thus, a refined detection or determination of the striker angle Sa resulting in the presence of the visual damage indicator is possible. As the striker angle Sa relates to the kinetic energy of the striker 110 impacting the tire 1, the kinetic energy resulting in the presence of the visual damage indicator is determined.

Figure 4b shows the tire 1 and the striker pendulum 100 from a different perspective, using the same reference signs as in Figure 4a where applicable. As visible in Figure 4b, the striker 110 has a camber angle (CA) with the axial direction a of the tire 1, which is preferably within a range of 2° and 20°, preferably between 3° and 10°, or even more preferably, within a range of 5° to 7°. The striker 110 may comprise a wedge shape and mechanically impact the shoulder region 7 with its wedge tip, or, in other words, edge.

An embodiment of a striker pendulum, particularly also including its arrangement and/or construction is described in further detail in standard GB/T 38528-2020. In this standard, the striker pendulum is also deflectable by a striker angle (Sa) and released to mechanically impact a circumferential sector of a shoulder region of the tire, similarly to the schematic representation of Figures 4a and 4b. A camber angle (CA) of the striker and, optionally, a slip angle of the striker are also defined in the standard. The kinetic energy described herein may also be considered as the drop energy according to the standard. Moreover, the energy of the striker creating a bulge exceeding 1 mm can also be described as the impact bulge energy according to the standard. Standard GB/T 38528-2020 is herewith incorporated by reference and may optionally be used.

The present invention is not limited to one specific visual damage indicator, which can be chosen or defined as deemed suitable as measure for observing and/or tracking tire damages caused by a mechanical impact in the shoulder region of the tire. Preferably, the visual damage indicator is defined as a bulge having a height of more than 1 mm (based on the original, and/or non-impacted tire surface, measured perpendicular to the surface area at which the bulge appears). Upon a certain magnitude of mechanical impact on the tire shoulder, the tire shoulder region and/or the tire's sidewall region may form such a bulge which can be detected.

Detection means for visual damage indicators include but are not limited to gauge dials, laser scanners or cameras. Upon, e.g., gradually, increasing the magnitude of an impact in different circumferential sectors of the shoulder region of each of the tires, such as the above mentioned three tires, each tire is typically associated with a different magnitude of mechanical impact at which the visual image indicator (such as a more than 1 mm high bulge) can be observed. In principle, it is possible to use the magnitude of mechanical impact at which the visual damage indicator is observed for the first time for further calculation and/or method steps, wherein first time may be considered herein as the lowest magnitude of mechanical impact resulting in the presence of the visual damage indicator. However, preferably, one or more further mechanical impacts are carried out (in further and/or separate circumferential sectors of the tire shoulder region), preferably with smaller changes/increments of magnitude than an earlier change/increment of magnitude between the mechanical impact causing the presence of the visual damage indicator and a preceding mechanical impact. Thereby, a more precise determination of the magnitude of the measure of the mechanical impact causing a presence of the visual damage indicator can be obtained. Moreover, it is also possible to carry out such measurements multiple times to obtain average values of the magnitude of the measure of the mechanical impact. Another option is to build, with the same type of fabric, multiple tires and test these tires having the same tire construction including the same type of fabric so as to obtain even more measurement values for determining the magnitude of the measure of the mechanical impact causing the presence of the visual damage indicator. In still another option, it is possible to form an average of i) the magnitude of the measure of the mechanical impact which has caused for the first time the presence of the visual damage indicator with ii) a magnitude of the measure of the mechanical impact causing the presence of the visual damage indicator based on refined (and/or smaller) changes or increases of magnitudes of mechanical impacts (e.g., from circumferential sector to circumferential sector). Such determinations, optionally including averaging and/or carrying out refined measurements or determinations of magnitudes of measures of mechanical impacts, shall also be covered by the present invention and its embodiments.

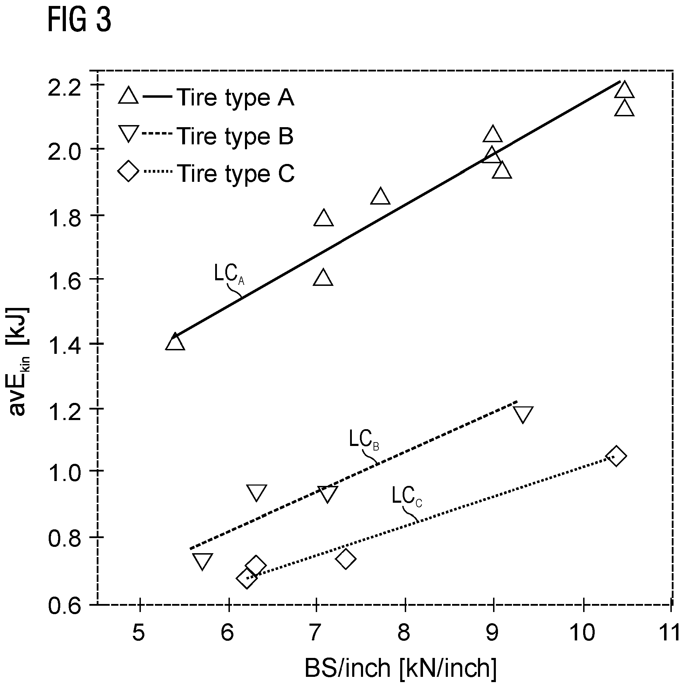

Figure 3 shows a diagram with three graphs representing three different tire types, i.e., tire type A, tire type B, and tire type C. Tire type A is built with nine different types of carcass fabrics resulting in nine tires differing in their respective type of carcass fabric and different measurement points for tire type A. In particular, the breaking strength per 2.54 cm (BS/2.54 cm) is determined for each fabric type of the nine different types of carcass fabrics. Furthermore, mechanical impacts of a striker pendulum with gradually increasing magnitude are carried out, and kinetic energies (avEₖᵢₙ) of the striker pendulum are determined for each tire having a different fabric type. In particular, such kinetic energy values are determined for the mechanical impacts of lowest magnitude of mechanical impact resulting in the presence or observation of a bulge having a height of more than 1 mm. Nine value pairs of such kinetic energy values and the corresponding breaking strengths per 2.54 cm of the respective fabric are obtained and plotted in the diagram for tire type A. A linear correlation of these values is shown in the form of the linear function LC_{A}. A coefficient of determination (R²) for this correlation is 93%. While not shown in Figure 3, it is also an option to extrapolate the linear function beyond the lowest determined breaking strength per 2.54 cm value and the highest breaking strength per 2.54 cm value determined.

Similarly to tire type A, again different types of fabrics are provided for tire type B, in this case four different types of fabrics. For each fabric a breaking strength per 2.54 cm is determined and tire type B is built four times, wherein each tire comprises one of the four types of fabrics. Furthermore, the kinetic energies avEₖᵢₙ, resulting in the presence or observation of a bulge having a height of more than 1mm, are determined for each tire, upon gradually increasing mechanical impact strength as described already above. The mechanical impacts of lowest magnitude resulting in the appearance of a bulge having a height of more than 1 mm (or an average of such values of multiple measurements) results together with the determined fabric breaking strength in one measurement point or value pair for tire type B. A linear correlation of the respective four value pairs for tire type B results in the dashed line LC_{B}. R² is at about 90% for LC_{B}. For a third tire type, i.e., tire type C, average kinetic energy values and breaking strengths per 2.54 cm of fabrics are determined in the same manner as for tire types A and B, and a linear correlation provides the linear function LCc, shown herein as dotted line. For this correlation, R² is about 97%.

While the breaking strength per 2.54 cm (e.g., in N/2.54 cm) has been chosen as a measure of a strength of the fabrics, the inventors have also carried out tests with the properties elongation at break (e.g., in percent of elongation at break) and work to break (e.g., in Nm/2.54 cm, according to ASTM D885, or equivalent). However, these two properties provide less favorable R² values, depending on the tire sample, such as within ranges of 15% to 66%, whereas the R² values for the breaking strength have been found to be within a considerably higher range from 84% to 99%.

In yet another option, it is possible to provide a threshold value of a kinetic energy which the tire must withstand without resulting in the presence of a bulge having a height of more than 1 mm. For instance, such a threshold energy may be the minimum breaking energy defined in said standard. Thus, it is possible to predict by means of the linear correlation, or correlation function, and the knowledge of the breaking strength per 2.54 cm of the respective fabric whether a certain tire type (for which the linear correlation has been determined) having this fabric as carcass fabric would pass / exceed the minimum breaking energy. Exceeding the minimum breaking energy can be associated to or understood as passing a test of tire impact resistance, particularly the test according to said standard. While this prediction may not be absolutely accurate in each single case, it may still be very helpful to expedite a tire development process and at least reduce the number of tires built and tested, particularly with regard to the choice of different types of carcass plies and/or carcass fabrics.

An additional advantage of the present invention is that it is possible to provide other types of fabrics which have not been used as carcass fabric in one of the built tire types (such as the tire types A, B, and C). Once the breaking strengths per 2.54 cm of the other types of fabrics are known, the determined linear correlation for the tire type of interest (such as function LC_{A}, LC_{B}, or LCc) can be used to estimate a corresponding magnitude of the measure of mechanical impact, e.g., a corresponding kinetic energy value (such as avEₖᵢₙ), which is deemed to cause the presence of a visual damage indicator (such as a bulge of more than 1 mm height). Thus, a link can be established between an either known, or easily determinable fabric property and a tire robustness indicator, i.e., said magnitude causing the presence of the visual damage indicator (such as said bulge).

It is emphasized that all aspects, their embodiments and features thereof can be combined with one another.

## Claims

1. A method of correlating fabric properties with tire properties comprising the steps of:
providing a set of different types of fabrics;
determining a magnitude of a measure of a strength of each type of fabric of the set of different types of fabrics;
providing a plurality of tires (1), wherein each tire of the plurality of tires comprises one type of fabric of the set of different types of fabrics as carcass fabric (3) of the tire (1);
carrying out mechanical impacts of different magnitude on the surface of a shoulder region (7) of each tire of the plurality of tires (1);
checking each tire of the plurality of tires (1) for a presence of a visual damage indicator at an outer surface of one or more of a sidewall region (6) and the shoulder region (7) of the tire upon carrying out one of the mechanical impacts;
determining for each tire of the plurality of tires (1) a magnitude of a measure of a mechanical impact causing the presence of the visual damage indicator; and
assigning the magnitude of the measure of the mechanical impact causing the presence of the visual damage indicator of each tire of the plurality of tires to the magnitude of the measure of the strength of the type of fabric comprised in the respective tire, so as to obtain for the set of different types of fabrics value pairs comprising the magnitude of the measure of the strength of one type of fabric and the magnitude of the measure of the mechanical impact causing the presence of the visual damage indicator for the tire comprising said one type of fabric.

2. The method of claim 1 further comprising the step of determining a correlation based on the value pairs.

3. The method according to claim 1 or 2, wherein carrying out the mechanical impacts includes:
mechanically impacting the tire in a first circumferential sector of its shoulder region (7) with a first magnitude of mechanical impact;
mechanically impacting the tire in a second circumferential sector of its shoulder region (7) with a second magnitude of mechanical impact;
wherein the second magnitude of mechanical impact is higher than the first magnitude of mechanical impact.

4. The method of claim 3, further comprising mechanically impacting the tire in one or more further circumferential sectors of its shoulder region (7) with one or more higher magnitudes of mechanical impact, gradually increasing from circumferential sector to circumferential sector until the presence of the visual damage indicator is observed in one or more of the sidewall region (6) and the shoulder region (7) of the tire.

5. The method according to at least one of the previous claims, wherein the visual damage indicator is one or more of a bulge, bulge having a height of more than 1 mm, a crack, an exposed cord, an exposed portion of the carcass fabric, and an open splice; and/or wherein the measure of the strength is a breaking strength.

6. The method according to at least one of the previous claims, wherein the magnitude of a measure of a mechanical impact causing the presence of the visual damage indicator comprises one of a kinetic energy value and a parameter representative of a kinetic energy of a mechanical impact means having mechanically impacted the tire.

7. The method according to at least one of the previous claims, wherein the correlation is a linear correlation, optionally comprising one or more of a linear fit, a linear regression, a linear interpolation, and a linear extrapolation.

8. The method according to at least one of the previous claims, wherein the mechanical impacts are carried out by a striker pendulum.

9. The method according to at least one of the previous claims, wherein each tire of the plurality of tires (1) is one or more of:
a mono carcass ply tire;
a tire comprising a carcass ply which comprises a rubber composition reinforced by one type of the fabrics; and
a tire having essentially the same construction as other tires of the plurality of tires, apart from a different type of fabric of the set of different types of fabrics as the carcass fabric.

10. The method according to at least one of the previous claims, wherein the fabrics (3) comprise one or more of:
a plurality of parallel textile cords; and
one or more of polyester cords, polyamide cords, glass-fiber cords, carbon-fiber cords, and cords comprising plant-based material.

11. The method according to at least one of the previous claims, wherein the method comprises:
providing at least three different types of textile fabrics;
determining, as the magnitude of the measure of the strength, a breaking strength of each textile fabric of the at least three different types of textile fabrics;
providing at least three tires (1), wherein each tire of the at least three tires comprises a carcass ply comprising one type of textile fabric of the set of at least three different types of textile fabrics;
providing for each tire, as the magnitude of the measure of the mechanical impact causing the presence of the visual damage indicator, one of a kinetic energy value of a mechanical impact means, and a parameter representative of a kinetic energy value of the mechanical impact means, which results in an observation of the visual damage indicator, and wherein the visual damage indicator is a bulge of more than 1 mm; and
determining a linear correlation between i) breaking strengths of the at least three types of textile fabrics and ii) magnitudes of the measure of the mechanical impacts causing the presence of the visual damage indicators for the at least three tires, based on three obtained value pairs.

12. The method according to claim 11, wherein at least two of the breaking strengths differ by at least 1000 N/2.54 cm.

13. The method according to at least one of the previous claims, wherein the visual damage indicator is observed by one or more of: by a laser scanning device, by a camera, by a dial gauge, and by eye.

14. The method according to at least one of the previous claims, further comprising the steps of:
providing a further type of fabric, which is different from the types of fabrics of the set of different types of fabrics;
determining a magnitude of the measure of the strength of the further type of fabric; and
estimating a magnitude of a measure of a mechanical impact causing the presence of a visual damage indicator based on the determined magnitude of the measure of the strength of the further type of fabric and the correlation.

15. The method according to at least one of the previous claims, further comprising the steps of:
providing a threshold value for the magnitude of the measure of the mechanical impact; and
checking whether the estimated magnitude is higher than the threshold value.
